# EUROPÄISCHE PATENTSCHRIFT

(11) **EP 2 601 975 B1**
(45) Veröffentlichungstag und Bekanntmachung des Hinweises auf die Patenterteilung: **17.12.2014**
(21) Anmeldenummer: 12194888.9
(22) Anmeldetag: 29.11.2012
(51) Int. Cl.: A61L 2/08, B65B 55/02, B67C 7/00, H01J 33/02, H01J 37/30

(54) **Vorrichtung und Verfahren zur Sterilisation von Innenwandungen von Behältnissen mit einer Reflektorvorrichtung für Elektronenstrahlung**
Device and method for sterilization of the internal walls of containers with a reflector device for e-beam
Dispositif et procédé de stérilisation de parois intérieures de récipients à l'aide d'un dispositif réflecteur pour radiation électronique

(30) Priorität: 08.12.2011 DE 102011056162
(43) Veröffentlichungstag der Anmeldung: 12.06.2013
(73) Patentinhaber: Krones AG, 93073 Neutraubling (DE)
(72) Erfinder: Knott, Josef, 93073 Neutraubling (DE); Krüger, Jochen, 93073 Neutraubling (DE)
(74) Vertreter: Bittner, Bernhard

(56) Entgegenhaltungen:
- EP-A1- 2 161 202
- WO-A2-2007/095205
- WO-A2-2008/129397
- US-A1- 2007 283 667
- US-B1- 6 509 127

## Beschreibung

Die vorliegende Erfindung bezieht sich auf eine Vorrichtung sowie auf ein Verfahren zum Sterilisieren von Innenwandungen von Behältnissen mit zumindest einem Elektronenstrahlemitter gemäß den Oberbegriffen der Ansprüche 1 und 8.

Vorrichtungen zum Sterilisieren von Behältnissen sind beispielsweise in Produktions- und Abfüllanlagen von Behältnis- bzw. Flaschenherstellern integriert, um vorzugsweise eine Sterilisation des Innenraumes der Behältnisse zu ermöglichen, welcher mit dem in das Behältnis aufzunehmenden Medium in Kontakt gebracht wird.

EP2161202, WO2008129397 offenbaren je eine Vorrichtung und ein Verfahren zum Sterilisieren von Behältnissen und insbesondere ein Verfahren und eine Vorrichtung zum Sterilisieren von Behältnissen mittels Elektronenstrahlen, wobei ein Strahlfinger in die Vorformlinge eingeführt wird.

Eine Sterilisation der Behältnisse kann dabei mittels unterschiedlichsten Methoden realisiert werden. So kann beispielsweise ein flüssiges bzw. fließfähiges Sterilisationsmedium (chemische Sterilisation) auf die Innenwandung des Behältnisses aufgebracht werden bzw. das Behältnis kann in ein derartiges Medium eingetaucht werden, wobei jedoch das Behältnis nach der Sterilisation zumeist noch gespült und getrocknet werden muss, um ein negatives Zusammenwirken zwischen dem Sterilisationsmedium und dem Füllmedium zu vermeiden.

Ebenso ist die Dampf- oder Heißluftsterilisation bekannt, d.h. die Sterilisation durch Erhitzen, wobei jedoch die zu sterilisierenden Behältnisse auf enorm hohe Temperaturen erhitzt werden müssen, um eine im Wesentlichen vollständige Sterilisation der Behältnisse durch Absterben der Bakterien zu ermöglichen. Dies könnte auch zur Deformation und nachhaltigen Beschädigung der zu sterilisierenden Behältnisse führen, so dass ein derartiges Verfahren insbesondere im Bereich der Herstellung von Kunststoffbehältnissen kaum Anwendung finden wird.

Auch die Sterilisation mittels Strahlung ist ein Verfahren, welches vorzugsweise bei bereits in ihre Endform expandierten Behältnissen eingesetzt wird, um somit eine vollständige Sterilisation zu erreichen. Als Strahlung werden dabei vorzugsweise durch einen Elektronenstrahlemitter emittierte Elektronenstrahlen verwendet, die in den Innenraum des Behältnisses eingebracht werden.

So offenbart beispielsweise auch DE 10 2008 045 187 A1 ein Verfahren zum Sterilisieren von Behältnissen, bei welchem ein Behandlungskopf in das Innere der geblasenen Flasche eingebracht wird, Strahlung emittiert und auf die innere Oberfläche der Flasche aufbringt. Dieser Behandlungskopf bzw. Strahlfinger ist von seiner Bauart derart ausgelegt, dass bereits in ihre Endgröße und -form expandierte Behältnisse, wie Flaschen etc. sterilisiert werden können, welche zudem zumindest einen definierten Flaschenmündungsdurchmesser sowie einen definierten Wandungsdurchmesser aufweisen, um dem Behandlungskopf bzw. dessen distalem Ende ein Eindringen in das Behältnisinnere sowie eine vollumfängliche Bestrahlung der inneren Oberfläche zur Sterilisation zu ermöglichen.

Derartige Behandlungsköpfe weisen jedoch funktionsbedingt einen Mindestaußendurchmesser von ca. 16mm auf und können damit zwar durch die meisten Behältnismündungen, jedoch nicht in klassische Vorformlinge eingeführt werden, da sich der Innendurchmesser dieser Vorformlinge von der Mündung aus zum distalen Ende hin betrachtet, verringert.

D.h., sofern anstatt der Sterilisation bzw. Bestrahlung von bereits vollständig ausgeformten Behältnissen bzw. Flaschen vorzugsweise Vorformlinge bzw. Preforms bestrahlt und damit sterilisiert werden sollen, würde bei einem Einsatz eines derartigen Behandlungskopfes und dessen Einführen in den Mündungsbereich des Vorformlings kurz hinter dessen Mündung eine sehr große Strahlungsdosis auf die Oberfläche des Innenraumes des Vorformlings auftreffen, während auf die innere Oberfläche des distalen, d.h. des unteren geschlossenen Endes des Vorformlings demgegenüber eine zu geringe Strahlungsdosis appliziert werden würde, wodurch eine vollumfängliche und prozesssichere Sterilisation der Innenwandung des Vorformlings nicht mehr gegeben wäre.

Demnach ist es die Aufgabe der vorliegenden Erfindung eine Vorrichtung und ein Verfahren zum Sterilisieren von Innenwandungen von Behältnissen und insbesondere von Vorformlingen zur Verfügung zu stellen, welche nicht zu sterilisierende Bereiche des Behältnisses abschattet und die auf den Mündungsbereich überdosiert auftreffende Strahlung derart bis zu den unteren, distalen Bereichen des Behältnisses weitergeführt wird, dass folglich eine im Wesentlichen gleichmäßige Bestrahlung des gesamten Innenraumes des Behältnisses gewährleistet werden kann.

Diese Aufgabe löst die vorliegende Erfindung mittels einer Vorrichtung gemäß den unabhängigen Ansprüchen.

Vorteilhafte Ausführungsformen und Weiterbildungen sind Gegenstand der Unteransprüche.

Folglich wird eine Vorrichtung zum Sterilisieren von Innenwandungen von Behältnissen mit zumindest einem Elektronenstrahlemitter mit wenigstens einem Elektronenstrahlbeschleuniger und mit einem Austrittsfenster für die Elektronenstrahlen, einer Transporteinrichtung zum Transportieren der zu sterilisierenden Behältnisse und einer Hubeinrichtung zur Ermöglichung einer Relativbewegung zwischen den Behältnissen und dem Austrittsfenster in einer Längsrichtung der Behältnisse beansprucht.

Diese erfindungsgemäße Vorrichtung weist weiterhin eine Reflektorvorrichtung auf, welche zumindest abschnittsweise formschlüssig und/oder reibschlüssig in einem Bereich des Austrittfensters mit dem Elektronenstrahlemitter verbunden ist und zumindest abschnittsweise während eines definierten Zeitraumes in einen Innenraum des zu sterilisierenden Behältnisses einbringbar ist, um die Elektronenstrahlen auf die Innenwandungen des Behältnisses zu applizieren. Vorzugsweise ist die Reflektorvorrichtung abnehmbar mit dem Elektronenstrahlemitter verbunden.

Wie aus dem allgemeinen Stand der Technik bekannt, werden bei einem Elektronenstrahlemitter die Elektronen von einer geeigneten Kathode emittiert und durch eine konstante elektrische Potentialdifferenz auf eine Anode zu beschleunigt. Die Freisetzung der im Kathodenmaterial vorhandenen Elektronen findet entweder durch den Prozess der Glühemission (Edison-Effekt), der Feldemission oder auch der Photoemission (äußerer Photoelektrischer Effekt) statt.

Die emittierten bzw. erzeugten Elektronenstrahlen werden also beschleunigt durch den Elektronenstrahlbeschleuniger über ein entsprechendes Austrittsfenster an die Umgebung abgegeben, um folglich auf ein entsprechend zu bestrahlendes Objekt auftreffen zu können. D.h., dass die Elektronen als Elektronenstrahl durch ein Austrittsfenster aus einer Vakuumkammer heraus beschleunigt werden, wobei das Austrittsfenster zumeist aus einer Metallfolie gebildet ist. Diese Metallfolie des Austrittsfensters wird allgemein aus einem Material hoher Festigkeit, wie beispielsweise aus Titan gebildet, um dem Druckunterschied zwischen dem Innenraum und der Außenseite der Vakuumkammer zu widerstehen.

Neben der Sterilisationseinrichtung, welche u.a. den Elektronenstrahlemitter aufweist, weist die erfindungsgemäße Vorrichtung ebenfalls eine Transporteinrichtung zum Transportieren der Behältnisse durch den Sterilisationsbereich der gesamten Anlage, welche u.a. zum Herstellen, Expandieren, Sterilisieren, Befüllen und Verschließen der Behältnisse dient, auf. Diese Transporteinrichtung bzw. Transporteinrichtungen können beispielsweise Transportsterne, Transportschnecken und/oder Transportbahnen etc. mit entsprechenden Greifelementen zum Greifen der Behältnisse im Mündungsbereich oder Tragelementen zum Tragen der Behältnisse im Bodenbereich aufweisen.

Eine weitere Einheit der erfindungsgemäßen Vorrichtung ist eine Hubeinrichtung, welche entweder die zu sterilisierenden Behältnisse von deren Transporteinrichtung in Richtung der Sterilisationseinrichtung und damit in Richtung des Elektronenstrahlemitters und dessen Austrittsfenster bewegt oder welche die besagte Sterilisationseinrichtung bzw. den Elektronenstrahlemitter in Richtung des zu sterilisierenden Behältnisses bewegt. Dabei werden die Behältnisse oder auch die Sterilisationseinrichtung im Wesentlichen in Längsrichtung der Behältnisse, d.h. in vertikaler Richtung bewegt. Infolgedessen führt die Sterilisationseinrichtung bzw. der Elektronenstrahlemitter eine Relativbewegung zu den Behältnissen bzw. von den Behältnissen weg oder auch die Behältnisse führen eine Relativbewegung zu der Sterilisationseinrichtung bzw. dem Elektronenstrahlemitter bzw. von diesem Weg aus.

Es ist jedoch auch denkbar, dass sowohl die Behältnisse als auch die Sterilisationseinrichtung bzw. Elemente der Sterilisationseinrichtung, wie beispielsweise der Elektronenstrahlemitter im Wesentlichen aufeinander zu bewegt werden, so dass beide Elemente, d.h. sowohl die Sterilisationseinrichtung bzw. der Elektronenstrahlemitter und auch die Behältnisse gemeinsam eine Relativbewegung zueinander ausführen.

Vorzugsweise handelt es sich bei den zu sterilisierenden Behältnissen um Vorformlinge bzw. Preforms, deren Innendurchmesser im unteren Bereich, d.h. dem Mündungsbereich gegenüberliegenden Bereich, im Wesentlichen geringer ist, als im Mündungsbereich. D.h., dass sich der Durchmesser des Vorformlings ausgehend vom Mündungsbereich im Wesentlichen zumindest abschnittsweise verjüngt.

Um nun die durch den Elektronenstrahlemitter emittierten Strahlen derart in den Innbereich des Behältnisses einbringen zu können, dass die emittierten Strahlen im Wesentlichen gerichtet auf die innere Oberfläche des Behältnisses auftreffen können, um infolgedessen vorzugsweise die gesamte innere Oberfläche vollständig sterilisieren zu können, wird eine erfindungsgemäße Reflektorvorrichtung bzw. ein Kollimator bzw. ein Kollimatorrohr verwendet, welche formschlüssig und/oder reibschlüssige mit dem Elektronenstrahlemitter, insbesondere im Bereich des Austrittsfensters, verbunden ist, um die Elektronenstrahlung im Wesentlichen aufnehmen bzw. auffangen und diese in Richtung des Innenraumes des Behältnisses leiten bzw. transportieren zu können.

Eine formschlüssige Verbindung zwischen der Reflektorvorrichtung und dem Elektronenstrahlemitter kann beispielsweise mittels entsprechender Haltevorrichtungen, wie in Nuten eingreifenden Vorsprüngen ermöglicht werden, wobei beispielsweise die Außenseite des Elektronenstrahlemitters Nuten bzw. Einbuchtungen bzw. Ausnehmungen aufweist, in welche Vorsprünge bzw. Nasen etc. der Reflektorvorrichtung eingreifen können. Auch ist es demgegenüber denkbar, dass die Reflektorvorrichtung Nuten und der Elektronenstrahlemitter Vorsprünge aufweist, die ineinander greifen.

Auch ein Schraubverschluss zwischen dem Elektronenstrahlemitter und der Reflektorvorrichtung ist denkbar, wobei beispielsweise der Elektronenstrahlemitter ein Außengewinde aufweist, auf welches ein an der Reflektorvorrichtung geschnittenes bzw. gerolltes Innengewinde aufgeschraubt werden kann.

Eine reibschlüssige Verbindung zwischen dem Elektronenstrahlemitter und der Reflektorvorrichtung kann beispielsweise durch einen Pressverbund erzeugt werden, indem zumindest ein Abschnitt, d.h. ein Endabschnitt der Reflektorvorrichtung über den Umfang eines Abschnittes des Elektronenstrahlemitters, in welchem vorzugsweise das Austrittsfenster angeordnet ist, gestülpt bzw. aufgepresst wird. Dazu weist zumindest dieser eine Abschnitt der Reflektorvorrichtung ein im Wesentlichen zumindest geringfügig elastisch verformbares Material auf, welches zuerst leicht gedehnt werden kann, um über den Abschnitt des Elektronenstrahlemitters geschoben werden zu können und sich danach wieder in seinen ursprünglichen Zustand zurück formiert, wodurch ein Anpressen des einen Abschnittes der Reflektorvorrichtung an den einen Abschnitt des Elektronenstrahlemitters gewährleistet wird.

Es ist auch denkbar, dass die Verbindung zwischen dem Elektronenstrahlemitter und der Reflektorvorrichtung sowohl formschlüssig, als auch reibschlüssig erfolgt, um ein ungewolltes Ablösen der Reflektorvorrichtung von dem Elektronenstrahlemitter zu vermeiden.

Vorzugsweise ist die form- und/oder auch reibschlüssige Verbindung jederzeit auflösbar, so dass bei Bedarf die Reflektorvorrichtung von dem Elektronenstrahlemitter entfernt und durch beispielsweise eine andere Reflektorvorrichtung mit einem geringeren oder größeren Innendurchmesser ausgetauscht werden kann. Folglich ist es denkbar, dass für die unterschiedlichst gestalteten Behältnisse bzw. Vorformlinge auch unterschiedlichst gestaltetet Reflektorvorrichtungen verwendet werden können (Garnituren).

Diese Reflektorvorrichtung bzw. Reflektoreinrichtung kann folglich während der Weiterleitung der Elektronenstrahlung vom Elektronenstrahlemitter in Richtung des Innenraumes des Behältnisses selbst in den Innenraum des Behältnisses eingebracht werden, indem beispielsweise die Reflektorvorrichtung durch die Hubeinrichtung in im Wesentlichen vertikaler Richtung auf das Behältnis zu bewegt wird oder indem das zu sterilisierende Behältnis durch die Hubeinrichtung in im Wesentlichen vertikaler Richtung auf die Reflektorvorrichtung zu bewegt wird. So ist es auch möglich, dass durch die Hubeinrichtung die zu sterilisierenden Behältnisse genauso wie die Reflektorvorrichtung in im Wesentlichen vertikaler Richtung, welcher der Längsrichtung der Behältnisse entspricht, aufeinander zu bewegt werden.

Dabei werden die durch den Elektronenstrahlemitter emittierten Elektronenstrahlen im Wesentlichen derart durch die Reflektorvorrichtung geleitet, dass die Strahlung gerichtet, d.h. in im Wesentlichen definierter Art und Weise und in definierter Strahlungsdosierung, d.h. Menge, auf die innere Oberfläche des zu sterilisierenden Behältnisses aufgebracht werden kann.

Demzufolge ist die vorliegende Erfindung auch auf eine Reflektorvorrichtung gerichtet, welche die von einem Elektronenstrahlemitter emittierten Elektronen zumindest einmal an ihrer inneren Oberfläche, welche vorzugsweise ein Material mit einer großen Kernmasse aufweist, reflektiert und zu gebündelter Elektronenstrahlung mit geringem Durchmesser und geringer Streuung bündelt und folglich im Wesentlichen gerichtet an die Innenwandung eines Körpers und bevorzugt eines Behältnisses abgibt.

Vorzugsweise ist eine derartige Reflektorvorrichtung rohrförmig und weist zumindest eine erste Öffnung zum Einlassen der Elektronenstrahlen in die Reflektorvorrichtung und eine zweite Öffnung zum Auslassen der Elektronenstrahlen in den Innenraum der zu sterilisierenden Behältnisse sowie eine innere Oberfläche zum Reflektieren und/oder Bündeln der Elektronenstrahlen auf. Vorzugsweise ist ein Querschnitt der zweiten Öffnung geringer als ein Querschnitt der ersten Öffnung. Vorzugsweise weist die Reflektorvorrichtung einen kreisförmigen Querschnitt auf.

D.h., dass zumindest die innere Oberfläche der Reflektorvorrichtung bevorzugt zumindest teilweise ein Material mit einer großen Kernmasse bzw. Kernladungszahl aufweist, um die Elektronenstrahlen an ihrer inneren Oberfläche im Wesentlichen vollständig zu reflektieren und vorzugsweise auch zu bündeln, um gebündelte Elektronenstrahlen durch deren Reflektion definiert bzw. gerichtet auf die innere Oberfläche des zu sterilisierenden Behältnisses aufbringen zu können. Demzufolge soll durch die Verwendung eines derartigen Materials eine ungewollte Absorption der Elektronenstrahlen weitgehend vermieden werden.

Dabei ist das Material, welches zumindest teilweise eine große Kernmasse aufweisen soll, vorzugsweise ausgewählt aus einer Gruppe bestehend aus Wolfram, Tantal, Platin, Gold und/oder Materialen mit vergleichbaren chemischen und physikalischen Eigenschaften.

Beispielsweise können auf Goldoberflächen für Elektronen im 100keV-Bereich Reflexionsgrade bis zu 50% bei senkrechtem Einfall und bis zu 70% bei flachem Einfall der Elektroden auf die Reflektionsoberfläche erreicht werden.

Vorteilhaft ist es folglich möglich die Elektronen in eine definierte Richtung zu lenken bzw. zu reflektieren und auf einen im Wesentlichen gering streuenden Strahldurchmesser zu bündeln.

Vorteilhafter Weise weist die innere Oberfläche der Reflektorvorrichtung keine Kantenbereiche oder Sprünge bzw. Rillen etc. auf, so dass die Strahlung definiert in Richtung des Innenraumes des zu sterilisierenden Behältnisses reflektiert werden kann, ohne eine ungewollte Ablenkung zu erfahren.

In einer bevorzugten Ausführungsform weist zumindest ein erster Abschnitt der Reflektorvorrichtung, welcher in den Innenraum des Behältnisses einbringbar ist, einen kleineren Durchmesser auf, als ein zweiter Abschnitt der Reflektorvorrichtung, welcher mit dem Elektronenstrahlemitter verbunden ist.

Dies ist vorteilhaft, da der Elektronenstrahlemitter zumeist ein Austrittsfenster zum Ausgeben der Elektronenstrahlen aufweist, welches größer im Durchmesser ist, als der Innenraum des zu sterilisierenden Behältnisses.

Demzufolge soll in vorteilhafter Weise ein Übergangsbereich zwischen der Öffnung der Reflektorvorrichtung, welche mit dem Elektronenstrahlemitter verbunden ist und der Öffnung der Reflektorvorrichtung, welche in den Innenraum des zu sterilisierenden Behältnisses einbringbar ist, geschaffen werden, so dass zum Einen die emittierten Elektronenstrahlen vollständig in die Reflektorvorrichtung aufgenommen und innerhalb dieser weitergeleitet und folglich im Wesentlichen vollständig auf die zu sterilisierende Oberfläche des Behältnisses aufgebracht werden, ohne dass Elektronenstrahlen ausgeblendet oder von der zu sterilisierenden Oberfläche abgelenkt werden. D.h., dass ein hoher Wirkungsgrad der Sterilisationseinrichtung mittels der Reflektorvorrichtung erlangt werden soll.

Folglich weist bevorzugt zumindest ein dritter Abschnitt der Reflektorvorrichtung, ausgehend vom zweiten Abschnitt in Richtung des ersten Abschnittes betrachtet, einen sich im Durchmesser verjüngenden Bereich auf.

D.h., dass der Übergangsbereich zwischen der ersten Öffnung und der zweiten Öffnung der Reflektorvorrichtung einen sich im Durchmesser verjüngenden Abschnitt aufweist, um ein vollständiges Eindringen in den Innenraum des Behältnisses durch die Reflektorvorrichtung bzw. zumindest eines entsprechenden Bereiches dieser Reflektorvorrichtung zu ermöglichen.

In einer weiteren bevorzugten Ausführungsform dient die Reflektorvorrichtung zur Leitung eines Temperierluftstromes zum Temperieren und bevorzugt zum Kühlen des Behältnisses zumindest während des Sterilisationsvorganges.

Folglich ist vorzugsweise eine Temperierungsvorrichtung zum Temperieren der zu sterilisierenden Behältnisse zumindest während des Sterilisationsvorganges derart aktiviert, dass diese einen Temperierluftstrom in Richtung der Behältnisse abgibt.

Dieser Temperierluftstrom dient vorzugsweise zum Kühlen der Folie des Elektronen-Austrittsfensters des Elektronenstrahlemitters, wobei der Temperierluftstrom bevorzugt in dessen Durchflussmenge derart eingestellt ist, dass die Luft nach dem Passieren des Austrittsfensters auf eine definierte Temperatur aufgrund der von dem Austrittsfenster abgegebenen Wärmestrahlung erhitzt wurde, wobei bevorzugt der besagte Temperierluftstrom zudem eine Erwärmung bzw. ein Warmhalten des Behältnisses ermöglicht.

Es wäre jedoch auch denkbar, dass der Temperierluftstrom bereits auf eine definierte Temperatur vorgeheizt die Temperierungsvorrichtung verlässt, wobei die Temperatur des Temperierluftstromes derart eingestellt ist, dass folglich eine Kühlung des Austrittsfensters und ebenso eine Temperierung des Behältnisses ermöglicht werden kann.

D.h., dass die Temperierungsvorrichtung beispielsweise im Bereich der Sterilisationsvorrichtung und vorzugsweise im Bereich des Elektrodenstrahlemitters angeordnet ist, um den Temperierluftstrom beispielsweise über das Austrittsfenster durch die Reflektorvorrichtung in den Innenraum des zu sterilisierenden Behältnisses einzubringen..

Des Weiteren wird ein Verfahren zum Sterilisieren von Innenwandungen eines Behältnisses mittels von einem Elektronenstrahlemitter abgegebenen Elektronenstrahlen beansprucht.

Gemäß dem erfindungsgemäßen Verfahren wird eine formschlüssig und/oder reibschlüssig in einem Bereich eines Austrittfensters der Elektronenstrahlen mit dem Elektronenstrahlemitter verbundene Reflektorvorrichtung zum Reflektieren und/oder Bündeln der Elektronenstrahlen zumindest abschnittsweise während eines definierten Zeitraumes in einen Innenraum des zu sterilisierenden Behältnisses eingebracht, um die Elektronenstrahlen auf die Innenwandungen des Behältnisses zu applizieren.

Gemäß einer bevorzugten Ausführungsform reflektiert die Reflektorvorrichtung zumindest einen der von dem Elektronenstrahlemitter emittierten Elektronenstrahlen mindestens einmal nach dessen Austritt aus dem Elektronenstrahlemitter über eine innere, zumindest teilweise ein Material mit einer großen Kernmasse aufweisende Oberfläche.

Folglich werden die Elektronen vorzugsweise an der inneren Oberfläche im Wesentlichen vollständig reflektiert und zu einem geringfügig streuenden Elektronenstrahl mit im Wesentlichen kleinem Durchmesser gebündelt, um in gerichteter Art und Weise auf die zu sterilisierende innere Oberfläche des Behältnisses aufgebracht bzw. appliziert werden zu können.

D.h., dass vorzugsweise mittels der Reflektorvorrichtung durch Applizieren von an einer inneren Oberfläche der Reflektorvorrichtung reflektierter und gebündelter Elektronenstrahlen der Innenraum von Behältnissen und insbesondere von Vorformlingen sterilisiert wird.

Dazu werden vorzugsweise, wie oben bereits beschrieben, mittels einer Hubeinrichtung zur Ermöglichung einer Relativbewegung zwischen den Behältnissen und der Reflektorvorrichtung, die Reflektorvorrichtung und/oder die Behältnisse während des definierten Zeitraumes wenigstens zeitweise mit einer relativen Bewegungsgeschwindigkeit in einer Längsrichtung der Behältnisse bewegt. D.h., dass entweder die Reflektorvorrichtung mittels der Hubeinrichtung in Richtung der zu sterilisierenden Behältnisse und/oder die zu sterilisierende Behältnisse mittels der Hubeinrichtung in Richtung der Reflektorvorrichtung bewegt werden.

Weitere Vorteile, Ziele und Eigenschaften der vorliegenden Erfindung werden anhand nachfolgender Beschreibung anliegender Zeichnung erläutert, in welcher beispielhaft eine Ausführungsform einer Reflektorvorrichtung einer erfindungsgemäßen Vorrichtung zum Sterilisieren eines Innenraumes von Behältnissen dargestellt wird.

Komponenten, welche in den Figuren wenigstens im Wesentlichen hinsichtlich ihrer Funktion übereinstimmen, können hierbei mit gleichen Bezugszeichen gekennzeichnet sein, wobei diese Komponenten nicht in allen Figuren gekennzeichnet und erläutert sein müssen.

In den Figuren zeigen:
- Fig. 1: eine Prinzipskizze einer aus dem Stand der Technik bekannten Sterilisationsvorrichtung zum Sterilisieren von Behältnissen mittels Elektronenstrahlen sowie eine Ausführungsform eines Vorformlings und das Auftreffen der Elektronenstrahlen auf diesen Vorformling;
- Fig.2: eine Prinzipskizze einer Ausführungsform einer Reflektorvorrichtung der erfindungsgemäßen Vorrichtung zum Sterilisieren eines Innenraumes von Behältnissen in einer außerhalb einer Ausführungsform eines Vorformlings befindlichen Positionierung; und
- Fig.3: eine Prinzipskizze einer Ausführungsform einer Reflektorvorrichtung der erfindungsgemäßen Vorrichtung zum Sterilisieren eines Innenraumes von Behältnissen gemäß der Fig.2 in einer zumindest teilweise im Innenraum einer Ausführungsform eines Vorformlings befindlichen Positionierung.

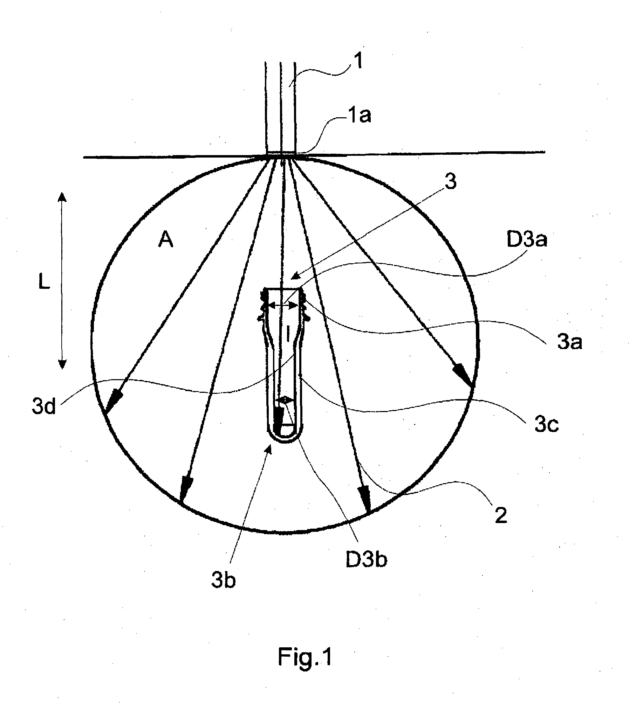

Fig.1 zeigt eine Prinzipskizze einer aus dem Stand der Technik bekannten Sterilisationsvorrichtung 1 bzw. einen Elektronenstrahlemitter 1 zum Sterilisieren von Behältnissen mittels Elektronenstrahlen 2 sowie eine Ausführungsform eines Vorformlings 3 und das Auftreffen der Elektronenstrahlen 2 auf diesen Vorformling 3.

Die gezeigte Ausführungsform des Vorformlings 3 weist einen Durchmesser D3a bzw. Innendurchmesser D3a im Mündungsbereich 3a auf, welcher größer ist, als der Durchmesser D3b bzw. Innendurchmesser D3b des unteren Bereiches 3b, so dass mittels eines aus dem bekannten Stand der Technik bekannten Strahlungsfinger keine vollständige Sterilisation zumindest des unteren Bereich 3b des Vorformlings 3, wie oben bereits erläutert, erfolgen würde.

Die von dem Elektronenstrahlemitter 1 emittierten Elektronenstrahlen 2 können ohne eine entsprechende Vorrichtung, wie der Reflektorvorrichtung, nur ungerichtet aus dem Austrittsfenster 1 a austreten und folglich teilweise zwar in den Innenbereich I bzw. Innenraum I des Vorformlings 3 gelangen oder auch nicht. Die emittierte Elektronenstrahlung 2 wird folglich auch in die Außenumgebung A des Vorformlings 3 abgegeben und dient demnach nicht mehr zur Sterilisierung der inneren Oberfläche 3d der Wandung 3c des Vorformlings 3. Die Strahlung 2 geht folglich ungenutzt verloren, wodurch zusätzliche Kosten entstehen.

Auch kann folglich keine vollumfängliche Sterilisation der Innenwandung 3d des Vorformlings 3 durch die Elektronenstrahlung 2 gewährleistet werden, da nicht vorhersehbar ist, wo die Elektronenstrahlen 2 genau eintreffen werden.

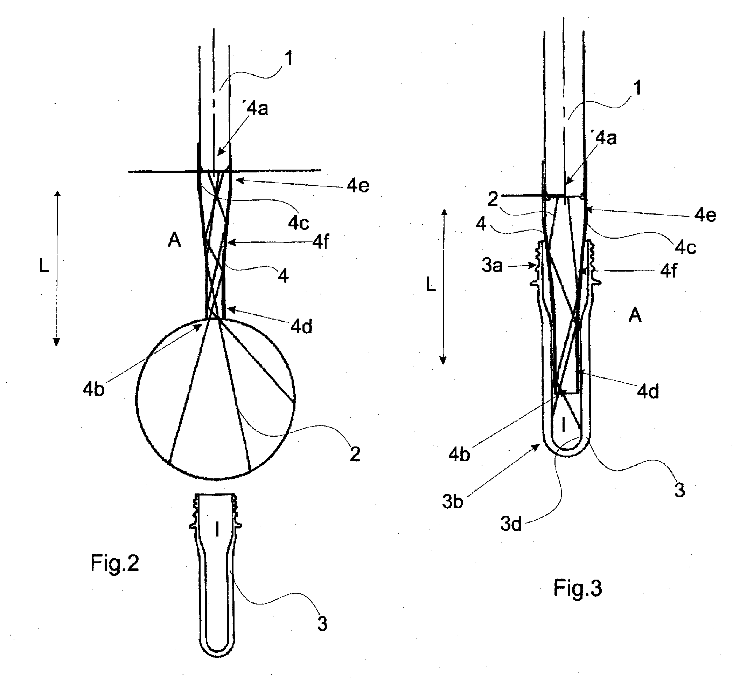

Um die Elektronenstrahlung 2 im Wesentlichen zu bündeln und auch gerichtet auf die innere Oberfläche 3d der Wandung 3c bzw. die Innenwandung 3c des Vorformlings 3 aufbringen zu können, wird erfindungsgemäß eine Reflektorvorrichtung 4 verwendet, die gemäß einer Ausführungsform als Prinzipskizze in einer außerhalb einer Ausführungsform eines Vorformlings 3 befindlichen Positionierung in Fig.2 dargestellt ist.

Die Reflektorvorrichtung 4 weist eine erste Öffnung 4a in einem zweiten Abschnitt 4e der Reflektorvorrichtung 4 auf, welche mit dem Elektronenstrahlemitter 1 und insbesondere mit dem Austrittsfenster 1a des Elektronenstrahlemitters 1 derart verbunden ist, dass die von dem Elektronenstrahlemitter 1 emittierten Elektronenstrahlen 2 über diese erste Öffnung 4a in die Reflektorvorrichtung 4 eindringen können, um von der Reflektorvorrichtung 4 in Richtung zu der zweiten Öffnung 4b, welche sich an einem ersten in den Vorformling 3 einbringbaren Abschnitt 4d der Reflektorvorrichtung 4 befindet, transportiert bzw. weitergeleitet zu werden.

Diese Weiterleitung erfolgt über eine Reflektion der Elektronenstrahlen 2 an der inneren Oberfläche 4c der Reflektorvorrichtung 4. Zudem werden die Elektronenstrahlen 2 durch diese innere Oberfläche 4c, welche vorzugsweise zumindest teilweise ein Material mit einer großen Kernmasse aufweist, nicht nur im Wesentlichen vollständig reflektiert, sondern auch zu Strahlen mit geringem Durchmesser gebündelt. Eine ungewollte Streuung der Elektronenstrahlen 2 soll damit weitestgehend vermieden werden.

Um ein Einbringen des ersten Abschnittes 4d der Reflektorvorrichtung 4 in den Innenraum I des Vorformlings 3 zu ermöglichen, ist der Durchmesser des ersten Abschnittes 4d im Wesentlichen kleiner bzw. geringer ausgeführt, als der Durchmesser des zweiten Abschnittes 4e der Reflektorvorrichtung 4, welcher vorzugsweise größer als der Durchmesser des ersten Abschnittes 4d gebildet ist, um eine form- und/oder reibschlüssige Verbindung mit dem Elektronenstrahlemitter 1 in der Weise zu ermöglichen, dass die Reflektorvorrichtung 4 beispielsweise im Wesentlichen zumindest abschnittsweise über den Elektronenstrahlemitter 1 gestülpt ist und somit einen Abschnitt des Elektronenstrahlemitters umfangsmäßig komplett umschließt. Dadurch können die von dem Elektronenstrahlemitter 1 emittierten Elektronenstrahlen 2 vollständig in die Reflektorvorrichtung 4 eingebracht werden, so dass keine Elektronenstrahlen 2 an die Außenumgebung A entweichen können und damit für die Sterilisation des Innenraumes I des Vorformlings 3 nicht mehr zur Verfügung stehen.

Infolgedessen weist die Reflektorvorrichtung 4 auch einen dritten Abschnitte 4f auf, welcher im Wesentlichen zwischen dem ersten Abschnitt 4d und dem zweiten Abschnitt 4e angeordnet ist und einen Durchmesser aufweist, der sich in Längsrichtung L der Reflektorvorrichtung 4, ausgehend vom zweiten Abschnitt 4e in Richtung des ersten Abschnittes 4d betrachtet, im Wesentlichen kontinuierlich verringert.

Die Fig.3 zeigt eine Prinzipskizze einer Ausführungsform einer Reflektorvorrichtung 4 der erfindungsgemäßen Vorrichtung zum Sterilisieren eines Innenraumes I von Behältnissen 3 gemäß der Fig.2 in einer zumindest teilweise im Innenraum I einer Ausführungsform eines Vorformlings 3 befindlichen Positionierung.

Demzufolge ist die Reflektorvorrichtung 4 und/oder auch der Vorformling 3 im Wesentlichen in Längsrichtung L mittels einer Hubeinrichtung (hier nicht gezeigt) derart bewegt worden, dass innerhalb eines definierten bzw. vorgegebenen Zeitraumes die Reflektorvorrichtung 4 zumindest mit dem ersten Abschnitt 4d im Wesentlichen kontinuierlich in den Innenraum I des Vorformlings eingebracht wird.

D.h., dass eine Relativbewegung vorzugsweise zwischen der Reflektorvorrichtung 4 und dem Vorformling 3 stattfindet, bei welcher entweder die Reflektorvorrichtung 4 auf den Vorformling 3 oder der Vorformling 3 auf die Reflektorvorrichtung 4 zubewegt wird. Jedoch ist es auch möglich, dass mittels der Hubeinrichtung sowohl die Reflektorvorrichtung 4 als auch der Vorformling 3 aufeinander zu bewegt werden, so dass zumindest der ersten Abschnitt 4d der Reflektorvorrichtung 4 in den Innenraum I des Vorformlings 3 im Wesentlichen schrittweise eingebracht werden kann.

Während des Vorganges des im Wesentlichen kontinuierlichen Einbringens bzw. Einfahrens des zumindest ersten Abschnittes 4d der Reflektorvorrichtung 4 in den Innenraum I des Vorformlings 3 werden Elektronenstrahlen 2 vom Elektronenstrahlemitter 1 emittiert, in die Reflektorvorrichtung 4 eingebracht, darin reflektiert und vorzugsweise auch gebündelt und als reflektierte und gebündelte Strahlen 2 auf die innere Oberfläche 3d des Vorformlings 3 aufgebracht.

Auch ist es möglich, dass während des Herausfahrens des zumindest ersten Abschnittes 4d der Reflektorvorrichtung 4 aus dem Innenraum I des Vorformlings 3 weiterhin Elektronenstrahlen 2 auf die innere Oberfläche 3d des Vorformlings zu deren Sterilisation aufgebracht werden, so dass eine Sterilisation des Innenraumes I des Vorformlings 3 vorzugsweise während der gesamten Relativbewegung der Reflektorvorrichtung 4 bzw. des Vorformlings 3 und insbesondere direkt nach Eintritt der Reflektorvorrichtung 4 in den Mündungsbereich 3a des Vorformlings 3 stattfindet.

### Bezugszeichenliste

- 1: Elektronenstrahlemitter
- 1a: Austrittsfenster
- 2: Elektronenstrahlen
- 3: Vorformling
- 3a: Mündungsbereich
- 3b: unteres/distales Ende
- 3c: Wandung
- 3d: innere Oberfläche/Innenwandung
- 4: Reflektorvorrichtung
- 4a: erste Öffnung
- 4b: zweite Öffnung
- 4c: innere Oberfläche
- 4d: erster Abschnitt
- 4e: zweiter Abschnitt
- 4f: dritter Abschnitt
- A: Außenumgebung
- D3a: Durchmesser im Mündungsbereich
- D3b: Durchmesser im unteren Bereich
- I: Innenraum
- L: Längsrichtung

## Patentansprüche

1. Vorrichtung zum Sterilisieren von Innenwandungen (3d) von Behältnissen (3) mit zumindest einem Elektronenstrahlemitter (1) mit wenigstens einem Elektronenstrahlbeschleuniger und mit einem Austrittsfenster (1a) für die Elektronenstrahlen (2), einer Transporteinrichtung zum Transportieren der zu sterilisierenden Behältnisse und einer Hubeinrichtung zur Ermöglichung einer Relativbewegung zwischen den Behältnissen (3) und dem Austrittsfenster (1a) in einer Längsrichtung (L) der Behältnisse (3),
**gekennzeichnet, durch**
eine rohrförmige Reflektorvorrichtung (4), welche zumindest eine erste Öffnung (4a) zum Einlassen der Elektronenstrahlen (2) in die Reflektorvorrichtung (4) und eine zweite Öffnung (4b) zum Auslassen der Elektronenstrahlen (2) in den Innenraum (I) der zu sterilisierenden Behältnisse (3) sowie eine innere Oberfläche (4c) zum Reflektieren und/oder Bündeln der Elektronenstrahlen (2) aufweist, wobei die Reflektorvorrichtung (4) zumindest abschnittsweise formschlüssig und/oder reibschlüssig mit seiner ersten Öffnung (4a) derart mit dem Austrittfenster (1 a) des Elektronenstrahlemitters (1) verbunden ist und zumindest abschnittsweise während eines definierten Zeitraumes in einen Innenraum (I) des zu sterilisierenden Behältnisses (3) einbringbar ist, um die Elektronenstrahlen (2) auf die Innenwandungen (3d) des Behältnisses (3) zu applizieren, dass die von dem Elektronenstrahlemitter (1) emittierten Elektronenstrahlen (2) über diese erste Öffnung (4a) in die Reflektorvorrichtung (4) eindringen können, um von der Reflektorvorrichtung (4) in Richtung zu der zweiten Öffnung (4b) weitergeleitet zu werden.

2. Vorrichtung nach Anspruch 1,
**dadurch gekennzeichnet, dass**
zumindest die innere Oberfläche (4c) der Reflektorvorrichtung (4) zumindest teilweise ein Material mit einer großen Kernmasse zur Reflexion von Elektronenstrahlen aufweist.

3. Vorrichtung nach Anspruch 2,
**dadurch gekennzeichnet, dass**
das Material ausgewählt ist aus einer Gruppe bestehend aus Wolfram, Tantal, Platin, Gold und/oder Materialen mit vergleichbaren chemischen und physikalischen Eigenschaften.

4. Vorrichtung nach einem der vorangegangenen Ansprüche,
**dadurch gekennzeichnet, dass**
zumindest ein erster Abschnitt (4d) der Reflektorvorrichtung (4), welcher in den Innenraum (I) des Behältnisses (3) einbringbar ist, einen kleineren Durchmesser aufweist, als ein zweiter Abschnitt (4e) der Reflektorvorrichtung (4), welcher mit dem Elektronenstrahlemitter (1) verbunden ist.

5. Vorrichtung nach Anspruch 4,
**dadurch gekennzeichnet, dass**
zumindest ein dritter Abschnitt (4f) der Reflektorvorrichtung (4), ausgehend vom zweiten Abschnitt (4e) in Richtung des ersten Abschnittes (4d), einen sich im Durchmesser verjüngenden Bereich aufweist.

6. Vorrichtung nach einem der vorangegangenen Ansprüche,
**dadurch gekennzeichnet, dass**
die Reflektorvorrichtung (4) zur Leitung eines Temperierluftstromes zum Temperieren des Behältnisses (3) während des Sterilisationsvorganges geeignet ist.

7. Vorrichtung nach einem der Ansprüche 1 bis 6,
**dadurch gekennzeichnet, dass**
die Behältnisse (3) Vorformlinge sind.

8. Verfahren zum Sterilisieren von Innenwandungen (3c) eines Behältnisses (3) mittels einer Vorrichtung gemäß zumindest einem der Ansprüche 1 bis 7, **dadurch gekennzeichnet, dass**
die formschlüssig und/oder reibschlüssig in einem Bereich eines Austrittfensters (1a) der Elektronenstrahlen (2) mit dem Elektronenstrahlemitter (1) verbundene Reflektorvorrichtung (4) zum Reflektieren und/oder Bündeln der Elektronenstrahlen (2) zumindest abschnittsweise während eines definierten Zeitraumes in einen Innenraum (I) des zu sterilisierenden Behältnisses (3) eingebracht wird, um die Elektronenstrahlen (2) auf die Innenwandungen (3c) des Behältnisses (3) zu applizieren.

9. Verfahren gemäß Anspruch 8,
**dadurch gekennzeichnet, dass**
die Reflektorvorrichtung (4) zumindest einen der von dem Elektronenstrahlemitter (1) emittierten Elektronenstrahlen (2) mindestens einmal nach dessen Austritt aus dem Elektronenstrahlemitter über eine innere, zumindest teilweise ein Material mit einer großen Kernmasse aufweisende Oberfläche (4c) reflektiert.

10. Verfahren gemäß Anspruch 8 oder 9,
**dadurch gekennzeichnet, dass**
mittels der Reflektorvorrichtung (4) durch Applizieren von an einer inneren Oberfläche (4c) der Reflektorvorrichtung (4) reflektierter und gebündelter Elektronenstrahlen (2) der Innenraum (I) von Vorformlingen (3) sterilisiert wird.

11. Verfahren gemäß einem der Ansprüche 8 bis 10,
**dadurch gekennzeichnet, dass**
mittels einer Hubeinrichtung zur Ermöglichung einer Relativbewegung zwischen den Behältnissen (3) und der Reflektorvorrichtung (4), die Reflektorvorrichtung (4) und/oder die Behältnisse (3) während des definierten Zeitraumes wenigstens zeitweise mit einer relativen Bewegungsgeschwindigkeit in einer Längsrichtung (L) der Behältnisse (3) bewegt werden.

## Claims

1. An apparatus for the sterilization of inner walls (3d) of containers (3) with at least one electron beam emitter (1) with at least one electron beam accelerator and with an outlet window (1 a) for the electron beams (2), a conveying device for conveying the containers to be sterilized and a lifting device for permitting a relative movement between the containers (3) and the outlet window (1 a) in a longitudinal direction (L) of the containers (3), **characterized by** a tube-shaped reflector apparatus, which comprises at least a first opening (4a) for the admittance of the electron beams (2) into the reflector apparatus (4) and a second opening (4b) for the exit of the electron beams (2) into the interior space (I) of the containers (3) to be sterilized and an inner surface (4c) for reflecting and/or bundling the electron beams (2), wherein the reflector apparatus (4) is connected at least in sections with its first opening (4a) in a positively locking and/or friction locking manner with the outlet window (1) of the electron beam emitter (1) in such a way and is capable of being introduced at least in sections during the defined time period in the interior space (I) of the container (3) to be sterilized in order to apply the electron beams (2) onto the inner walls (3d) of the container (3), so that the electron beams (2) emitted by the electron beam emitter (1) can penetrate into the reflector apparatus (4) by means of this first opening (4a) in order to be guided further in the direction to the second opening (4b) by the reflector apparatus.

2. An apparatus according to claim 1, **characterized in that** at least the internal surface (4c) of the reflector apparatus (4) has at least in part a material with a large nucleus mass for reflecting electron beams.

3. An apparatus according to claim 2, wherein the material is selected from a group consisting of tungsten, tantalum, platinum, gold and/or materials with comparable chemical and physical properties.

4. An apparatus according to one of the preceding claims, wherein at least one first portion (4d) of the reflector apparatus (4), which is capable of being introduced into the interior space (I) of the container (3), has a smaller diameter than a second portion (4e) of the reflector apparatus (4), which is connected to the electron beam emitter (1).

5. An apparatus according to claim 4, wherein at least one third portion (4f) of the reflector apparatus (4), as viewed in the direction of the first portion (4d) starting from the second portion (4e), has a region which tapers in diameter.

6. An apparatus according to one of the preceding claims, **characterized in that** the reflector apparatus (4) is appropriate for guiding a tempering air flow for tempering the container (3) during the sterilization procedure.

7. An apparatus according to one of the claims 1 to 6,
**characterized in that**
the containers (3) are preforms.

8. A method of sterilizing inner walls (3c) of a container (3) by means of an apparatus according to at least one of the claims 1 to 7, **characterized in that** a reflector apparatus (4) - connected in a region of an outlet window (1 a) of the electron beams (2) in a positively locking and/or friction locking manner to the electron beam emitter (1) - for reflecting and/or bundling the electron beams (2) is introduced at least locally during a defined period of time into an interior space (I) of the container (3) to be sterilized, in order to apply the electron beams (2) to the inner walls (3c) of the container (3).

9. A method according to claim 8, **characterized in that** the reflector apparatus (4) reflects at least one of the electron beams (2) emitted by the electron beam emitter (1) at least once after the exit thereof out of the electron beam emitter by way of an internal surface (4c) having at least in part a material with a large nucleus mass.

10. A method according to claims 8 or 9, **characterized in that** the interior space (I) of pre-forms (3) is sterilized by means of the reflector apparatus (4) by the application of electron beams (2) reflected and bundled on an internal surface (4c) of the reflector apparatus (4).

11. A method according to one of the claims 8 to 10, **characterized in that** the reflector apparatus (4) and/or the containers (3) is or are moved during a defined period of time at least for a time at a relative movement velocity in a longitudinal direction (L) of the containers (3) by means of a lifting device in order to permit a relative movement between the containers (3) and the reflector apparatus (4).

## Revendications

1. Dispositif pour la stérilisation de parois intérieures (3d) de récipients (3) comportant au moins un émetteur de rayonsrayons d'électrons (1) avec au moins un accélérateur d'électrons, et avec une fenêtre de sortie (1a) pour les rayons d'électrons (2), un dispositif de transport pour la transportation des récipients à stériliser et un dispositif de levage permettant un déplacement relatif entre les récipients (3) et la fenêtre de sortie (1 a) dans une direction longitudinale (L) des récipients (3),
**caractérisée par**
un dispositif réflecteur (4) tubulaire présentant au moins une première ouverture (4a) pour la pénétration des rayons d'électrons (2) dans le dispositif réflecteur (4) et une deuxième ouverture (4b) pour la sortie des rayons d'électrons (2) vers l'intérieur (I) des récipients (3) à stériliser, ainsi qu'une surface intérieure (4c) pour la réflexion et/ou la focalisation des rayons d'électrons (2),
le dispositif réflecteur (4) étant raccordé au moins en partie par correspondance de forme et/ou par friction à la fenêtre de sortie (1a) de l'émetteur de rayons d'électrons (1) par sa première ouverture (4a), et pouvant être introduit au moins en partie à l'intérieur (I) du récipient (3) à stériliser pendant une durée définie, pour appliquer les rayons d'électrons (2) contre les parois intérieures (3d) du récipient (3), de telle manière que les rayons d'électrons (2) émis par l'émetteur de rayons d'électrons (1) puissent pénétrer dans le dispositif réflecteur (4) par ladite première ouverture (4a) pour être transmis vers la deuxième ouverture (4b) par le dispositif réflecteur (4).

2. Dispositif selon la revendication 1, **caractérisée en ce qu'**
au moins la surface intérieure (4c) du dispositif réflecteur (4) présente au moins partiellement un matériau de masse nucléaire élevée pour refléter les rayons d'électrons.

3. Dispositif selon la revendication 2, **caractérisée en ce que**
le matériau est sélectionné dans un groupe consistant de tungstène, le tantale, le platine, l'or et/ou des matériaux présentant des propriétés chimiques et physiques comparables.

4. Dispositif selon l'une des revendications précédentes, **caractérisée en ce qu'**
au moins un premier segment (4d) du dispositif réflecteur (4) pouvant être introduit à l'intérieur (I) du récipient (3) présente un diamètre inférieur à celui d'un deuxième segment (4e) du dispositif réflecteur (4) relié à l'émetteur de rayons d'électrons (1).

5. Dispositif selon la revendication 4, **caractérisée en ce qu'**
au moins un troisième segment (4f) du dispositif réflecteur (4) partant du deuxième segment (4e) vers le premier segment (4d) présente une partie dont le diamètre se rétrécit.

6. Dispositif selon l'une des revendications précédentes, **caractérisée en ce que**
le dispositif réflecteur (4) est adapté pour conduire un flux d'air pour l'équilibrage de température du récipient (3) pendant le processus de stérilisation.

7. Dispositif selon l'une des revendications 1 à 6, **caractérisée en ce que**
les récipients (3) sont des préformes.

8. Procédé de stérilisation de parois intérieures (3c) d'un récipient (3) au moyen d'un dispositif selon au moins une des revendications 1 à 7, **caractérisé en ce que**
le dispositif réflecteur (4) raccordé au moins en partie par correspondance de forme et/ou par friction à l'émetteur de rayons d'électrons (1) dans une zone d'une fenêtre de sortie (1a) des rayons d'électrons (2), pour la réflexion et/ou la focalisation des rayons d'électrons (2), est introduit au moins en sections à l'intérieur (I) du récipient (3) à stériliser pendant une durée définie, pour appliquer les rayons d'électrons (2) contre les parois intérieures (3c) du récipient (3).

9. Procédé selon la revendication 8, **caractérisé en ce que**
le dispositif réflecteur (4) reflète au moins un des rayons d'électrons (2) émis par l'émetteur de rayons d'électrons (1), au moins une fois après la sortie de celui-ci hors de l'émetteur de rayons d'électrons, au moyen d'une surface intérieure (4c) présentant au moins partiellement un matériau de masse nucléaire élevée.

10. Procédé selon la revendication 8 ou 9, **caractérisé en ce que**
l'intérieur (I) de préformes (3) est stérilisé au moyen du dispositif réflecteur (4), par application de rayons d'électrons (2) reflétés et focalisés sur une surface intérieure (4c) du dispositif réflecteur (4).

11. Procédé selon l'une des revendications 8 à 10, **caractérisé en ce qu'**
au moyen d'un dispositif de levage permettant un déplacement relatif entre les récipients (3) et le dispositif réflecteur (4), le dispositif réflecteur (4) et/ou les récipients (3) sont déplacés dans une direction longitudinale (L) des récipients (3) pendant la durée définie, au moins temporairement avec une vitesse de déplacement relative.
